# EUROPEAN PATENT SPECIFICATION

(11) **EP 1 441 717 B1**
(45) Date of publication and mention of the grant of the patent: **08.08.2007**
(21) Application number: 02783345.8
(22) Date of filing: 25.10.2002
(51) Int. Cl.: A61K 31/245, A61K 31/495, A61P 35/02

(54) **COMBINATION OF AN ATP-COMPETITIVE INHIBITOR OF BCR/ABL KINASE ACTIVITY AND A TYRPHOSTIN ANALOG**
KOMBINATION EINES ATP-COMPETITIVEN INHIBITORS DER BCR/ABL KINASE UND EINES TYRPHOSTIN ANALOGEN
COMBINAISON D'UN INHIBITEUR ATP COMPETITIF D'ACTIVITE DE KINASE BCR/ABL ET ANALOGUE DE TYRPHOSTINE

(30) Priority: 30.10.2001 US 339032 P
(43) Date of publication of application: 04.08.2004
(73) Proprietor: MAYO FOUNDATION FOR MEDICAL EDUCATION AND RESEARCH, Rochester, MN 55905 (US)
(72) Inventor: KAUFMANN, Scott, H., Rochester, MN 55901 (US); MOW, Benjamin, Singapore 670170 (SG)
(74) Representative: de Weerd, Petrus G.W.
(86) International application number: PCT/IB2002/004430
(87) International publication number: WO 2003/037322

(56) References cited:
- CARLO-STELLA CARMELO ET AL: "Effects of the tyrosine kinase inhibitor AG957 and an anti-Fas receptor antibody on CD34+ chronic myelogenous leukemia progenitor cells." BLOOD, vol. 93, no. 11, 1 June 1999 (1999-06-01), pages 3973-3982, XP002231135 ISSN: 0006-4971 cited in the application
- DRUKER B J ET AL: "LESSONS LEARNED FROM THE DEVELOPMENT OF AN ABL TYROSINE KINASE INHIBITOR FOR CHRONIC MYELOGENOUS LEUKEMIA" JOURNAL OF CLINICAL INVESTIGATION, NEW YORK, NY, US, vol. 105, no. 1, January 2000 (2000-01), pages 3-7, XP001014734 ISSN: 0021-9738
- AKIN C ET AL: "EFFECTS OF THE TYROSINE-KINASE INHIBITOR STI571 ON MUTATED KIT AND NEOPLASTIC MAST CELLS" BLOOD, W.B.SAUNDERS COMPAGNY, ORLANDO, FL, US, vol. 96, no. 11, PART 1, 16 November 2000 (2000-11-16), page 747A XP001098218 ISSN: 0006-4971

## Description

The invention relates to a combination of (a) an ATP-competitive inhibitor of Bcr/abl kinase activity and (b) a tyrphostin analog, wherein the ATP-competitive inhibitor of bcr/abl kinase activity (a) is (N-{5-[4-(4-methyl-piperazino-methyl)-benzoylamido]-2-methylphenyl}-4-(3-pyridyl)-2-pyrimidine-amine, and the tyrphostin analog (b) is Adaphostin, or a pharmaceutically acceptable salt of any one of components (a) or (b) where salt-forming groups are present. a pharmaceutical preparation comprising (a) and (b) in combination together with a pharmaceutically acceptable carrier material; a product comprising (a) and (b) as defined above and optionally a pharmaceutically acceptable carrier material, for simultaneous, chronically staggered or separate use, or any combination thereof; a method of administering or the use of said combination or product for the treatment of bcr/abl-related diseases; and/or to the use of said combination or product for the manufacture of a medicament for the treatment of bcr/abl-related diseases.

### Background of the invention

Around 4500 new cases of CML are reported in the United States each year. In the vast majority of these cases, a characteristic t(9;22) translocation juxtaposes the 5' end of the bcr gene with the 3' end of the abl gene, resulting in a unique 210 kDa fusion protein p210^{bcr/abl2-5} This constitutively active cytoplasmic kinase is capable of not only transforming murine fibroblasts and hematopoietic cell lines, but also causing a chronic myeloproliferative disorder resembling CML upon transduction into mouse marrow. This p210^{bcr/abl}-induced transformation appears to involve activation of multiple signaling pathways, including the Ras-Raf pathway and the phosphatidylinositol-3 kinase/Akt pathway, as well as transcription mediated by signal transducer and activator of transcription 5 (Stat5) and nuclear factor κB. Collectively, these signal transduction events result in upregulation of antiapoptotic proteins, including Bcl-xL, the X-linked inhibitor of apoptosis protein (XIAP) and survivin, contributing to the resistance of p210 ^{bcr/abl}-expressing cells to a variety of apoptotic stimuli.

Until recently, treatment options for CML, which included the use of hydroxyurea, α-interferon with or without cytarabine or stem cell transplantation, were less than satisfactory because of patient intolerance or lack of effect on the natural history of this disorder. The presence of the p210^{bcr/abl} kinase in the vast majority of CML cases, coupled with evidence implicating this kinase in the pathogenesis of CML, made this fusion protein an attractive target for CML-directed therapy. Previous efforts identified multiple p210^{bcr/abl} kinase inhibitors.

The most widely studied p210^{bcr/abl} inhibitor is STI571 (formerly known as CGP 57148). The ATP-binding site-directed agent STI571 is in the meantime already being marketed, e.g. in the USA under the tradename Gleevec^{®}. This agent is a reversible inhibitor that occupies the ATP binding pocket of p21 ^{bcr/abl} and stabilizes the kinase in an inactive conformation. Preclinical studies demonstrated that STI571 also inhibits the kinase activities of c-abl, platelet-derived growth factor receptor and the c-kit receptor. Phase I studies showed that STI571 has impressive activity against chronic phase CML but more limited activity against p190^{bcr/abl}-expressing acute lymphocytic leukemia and the blast crisis phase of CML. Additional preclinical and clinical studies of STI571, alone and in combination with conventional cytotoxic agents, are currently ongoing.

An alternative approach to inhibiting protein kinases involves the use of small molecules that alter the binding of peptide substrates rather than ATP. A chemically diverse group of agents generically termed tyrphostins have been synthesized and evaluated as potential inhibitors of various tyrosine kinases (see Levitski, FASEB J. 6, 3275-3282 (1992)). The tyrphostin AG957 was previously found to inhibit p210^{bcr/abl} activity in immune complex kinase assays and to cause decreased p210^{bcr/abl} autophosphorylation followed by bcr/abl degradation in intact cells.

AG957 was found to also inhibit T cell receptor-mediated phosphorylation of the adaptor protein c-cbl, suggesting that kinases other than bcr/abl might also be affected. Despite the lack of absolute specificity for bcr/abl-transformed cell lines, AG957 was observed to selectively inhibit proliferation of CML progenitors as compared to normal myeloid progenitors (see Carlo-Stella et al., Blood 93, 3973-3982 (1999) and Svingen et al., Clin. Cancer Res. 6, 237-49 (2000)). Subsequent animal testing has revealed that AG957 has a very short serum half-life (S. Stinson, V.L. Narayanan and E. A. Sausville, unpublished observations). Examination of a series of AG957 analogues has identified the adamantyl ester adaphostin (NSC680410) as a derivative with higher potency *in vitro* (see Svingen et al., *loc. cit.)* and a longer serum half-life *in vivo* (S. Stinson, V.L. Narayanan and E. A. Sausville, unpublished observations). Adaphostin is among the tyrphostin derivatives that are currently undergoing evaluation as bcr/abl kinase inhibitor.

In view of the possibility of resistance development against any of the active ingredients, it remains a major goal to identify new treatment regimens that allow for optimal patient treatment, as well as identifying new methods of treatment.

### General Description of the invention

Surprisingly, it has been found that the combination of (a) an ATP-competitive inhibitor of Bcrl/abl kinase activity and (b) a tyrphostin analog each administered in sufficiently short time intervals to allow for mutual influence on efficiency are capable of even synergistic and/or non-cross-resistant effects in the treatment of bcr/abl-related diseases.

The present invention shows, for the first time, the tying together of the two strategies of causing beneficial effects with regard to bcr/abl-related diseases. The effect of a combination as defined herein is especially greater than the effects that can be achieved with either type of combination partner alone, i.e. greater than the effects of a monotherapy using only one of the combination partners (components) (a) and (b) as defined herein.

Under certain circumstances, drugs with totally different mechanisms of action may be combined. However, just considering any combination of drugs having different mode of action does not necessarily lead to combinations with advantageous effects.

All the more surprising is the effect of the combination of components (a) and (b) in the treatment of bcr/abl-related diseases. Both component (a) and (b) are inhibitors of Bcr/abl kinase activity; however, the results of the present invention suggest that they triggering different downstream events and that, in combination, they are capable of acting in a mutually enhancing way.

A further benefit is that lower doses of the active ingredients of the COMBINATION OF THE INVENTION as defined below can be used, for example, that the dosages need not only often be smaller but are also applied less frequently, or can be used in order to diminish the incidence of side-effects. This is in accordance with the desires and requirements of the patients to be treated.

The COMBINATION OF THE INVENTION especially shows a synergistic therapeutic effect, e.g. with regard to slowing down, arresting or reversing formation or a longer duration of bcr/abl-related diseases, but also in further surprising beneficial effects, e.g. allowing for less side-effects, an improved quality of life and a decreased mortality and morbidity, compared to a monotherapy applying only one of the pharmaceutically active ingredients used in the COMBINATION OF THE INVENTION.

### Detailed description of the invention

The invention relates to a combination of (a) an ATP-competitive inhibitor of Bcr/abl kinase activity and (b) a tyrphostin analog, wherein the ATP-competitive inhibitor of bcr/abl kinase activity (a) is (N-{5-[4-(4-methyl-piperazino-methyl)-benzoylamido]-2-methylphenyl}-4-(3-pyridyl)-2-pyrimidine-amine, and the tyrphostin analog (b) is Adaphostin, or a pharmaceutically acceptable salt of any one of components (a) or (b) where salt-forming groups are present.

In a further aspect, the invention relates to a pharmaceutical preparation comprising (a) and (b) as mentioned in the last paragraph in combination together with a pharmaceutically acceptable carrier material.

The invention also relates to a (commercial) product comprising (a) and (b) as defined above and optionally a pharmaceutically acceptable carrier material, for simultaneous, chronically staggered and/or separate use, or any combination thereof.

The invention also relates to the use of said combination of (a) and (b) or said product comprising (a) and (b) for the manufacture of a medicament for the treatment of a bcr/abl-proliferative disease.

In addition, the invention relates to component (a), as defined hereinabove or hereinbelow, for use in combination, that is at the same time point or in a chronologically staggered way, with a component (b) as defined hereinabove or hereinbelow, or vice versa a component (b) for use in combination with a component (a) as defined hereinabove or hereinbelow, especially in the treatment of a bcr/abl-related disease; especially said components in a packaging and with a description (e.g. package leaflet) suggesting such combination.

The general terms used hereinbefore and hereinafter preferably have within the context of this disclosure the following meanings, unless otherwise indicated:

Component (a): (N-{5-[4-(4-methyl-piperazino-methyl)-benzoylamido]-2-methylphenyl}-4-(3-pyridyl)-2-pyrimidine-amine, especially in the form of the methane sulfonate (monomesylate) salt (STI571) an ATP-competitive inhibitor of bcr/abl kinase activity especially of the p210^{bcr/abl} 210 kDa fusion protein, or a pharmaceutically acceptable salt thereof, as described in EP 0 564 409.

The inhibition of bcr/abl kinase can be determined according to methods known in the art (see, e.g., Nature Medicine 2, 561-566 (1996), or Gombacorti et al., Blood Cells, Molecules and Diseases 23, 380-394 (1997)).

Component (b): A tyrphostin adaphostin (4-{[(2,5-dihydroxyphenyl)methyl]amino}-benzoic acid adamantyl ester; NSC 680410) of the formula 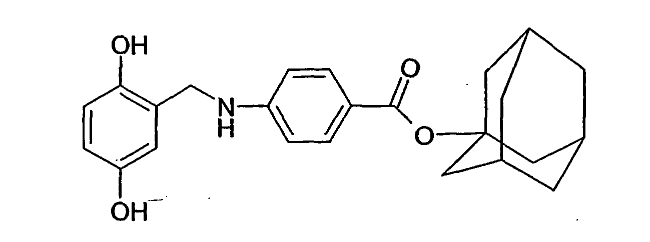 or a salt thereof.

The components each may be present in free form or in the form of (preferably pharmaceutically acceptable) salts, e.g. in the case of basic salt-forming groups, such as amino or imino or the hydroxy of a phenol, as acid addition salts, e.g. with organic or inorganic acids, for example chloride or mesylate salts, in case of presence of acidic groups (e.g. carboxy or sulfo) in the form of metal, e.g. alkaline metal, or quarternary ammonium salts, e.g. tert-butylammonium salts, or in case that basic and acidic goups are present in the form of inner or mixed salts, and reference to the components is always intended to mean the components in free and/or in salt form. The combination partner (a) or (b) or a pharmaceutically acceptable salt thereof may also be used in form of a hydrate or include other solvents used for crystallization. N-{5-[4-(4-methyl-piperazino-methyl)-benzoylamido]-2-methylphenyl}-4-(3-pyridyl)-2-pyrimidine-amine, as combination partner (a), is preferably used in the present invention in the form of its monomesylate salt (STI571).

A combination which comprises component (a), N-{5-[4-(4-methyl-piperazino-methyl)-benzoylamido]-2-methylphenyl}-4-(3-pyridyl)-2-pyrimidine-amine, and a component (b), adaphostin, in which the active ingredients are present independently of each other in free form or in the form of a (preferably pharmaceutically acceptable) salt and optionally at least one pharmaceutically acceptable carrier, and are together part of a combination preparation or part of a product with separate preparations (e.g. in the sense of a kit of parts) will be referred to herein as a COMBINATION OF THE INVENTION.

The combination partner (a) can be prepared and administered as described in EP 0 564 409, preferably as described in WO 99/03854, especially the monomesylate salt of N-{5-[4-(4-methyl-piperazino-methyl)-benzoylamido]-2-methylphenyl}-4-(3-pyridyl)-2-pyrimidine-amine can be formulated as described in Examples 4 and 6 of WO 99/03854.

In addition to components (a) and (b), combination with further antineoplastic agents is possible. The term "antineoplastic agents" as used herein includes, but is not limited to aromatase inhibitors, e.g. letrozole, antiestrogens, topoisomerase I inhibitors, topoisomerase II inhibitors, microtubule active agents, e.g. paclitaxel, taxotere or epothilone A or B, alkylating agents, e.g. cyclophosphamide, antineoplastic antimetabolites, platin compounds, compounds decreasing the protein kinase activity and further anti-angiogenic compounds, gonadorelin agonists, anti-androgens, bisphosphonates and trastuzumab.

Comprised are likewise the corresponding stereoisomers as well as the corresponding crystal modifications, e.g. solvates and polymorphs, of components (a) and (b), as well as further antineoplastic agents if present.

Bcr/abl-related diseases are especially bcr/abl-positive proliferative, especially cancer and tumor, diseases, especially leukemias, such as chronic-myleogenic leukemia (CML) and acute lymphoblastic leukemia (ALL), where especially an effect due to apoptosis is found, but also further gliomas, sarcomas, ovarial tumors, and the like, also showing effects on the subgroup of leukeimic stem cells and potential for the prurification of such cells in vitro after removal from the body (for example, removal of bone marrow) and subsequent re-implantation of cells purified from cancer cell (e.g. re-implantation of purified bone marrow cells).

The increase in efficiency of a combination of components (a) and (b) over the single components can be observed using standard procedures.

Especially in Example 1, methods are shown that allow the determination of the increase in apoptosis induction showing the increase in efficiency of the combination when compared with the single components - these methods can also be used for different components (a) and (b) than those given in the Examples.

Furthermore, animals can be used (e.g. pigs, minipigs, baboons, rats, rabbits and especially mice) to show the efficiency of the combination according to the invention in the treatment of bcr/abl-related diseases, especially corresponding proliferative diseases. For example, the antiproliferative, especially antitumor and anti-leukemia, activity of the combinations can be demonstrated using the following experiments: The in-vivo- antitumor activity is tested using the Balb/c AmuLV A6R1 (ATCC: TIB90), c-sis and v-sis transformed BALB/c 3T3 cell lines or the human entrogen dependent breast carcinoma MCF-7 (ATCC: HTB22) or ZR-75-1 (ATCC: CRL1500); the estrogen-independen breast carcinomas MDA-MB468 (ATCC: HTB132) or MDA-MB231 (ATCC: HTB26); the colon-carzinoma Colo 205 (ATCC: CCL222); the glioblastomas U-87MG (ATCC: HTB14) or U-373MG (ATCC: HTB17); the "small cell lung carcinomas" NCI-H69 (ATCC: HTB119) and NCI-H209 (ATCC: HTB172); the lung carcinoma NCI-H596 (ATCC: HTB178); the melanomas Hs294T (ATCC: HTB140) or A375 (ATCC: CRL1619); the ovarial carcinoma NIH-Ovcar3 (ATCC: HTB161); as well as the prostate carzinomas DU145 (ATCC: HTB81) or PC-3 (ATCC: CRL1435); the bladder carzinoma T24 (ATCC: HTB4); the epithelial carcinoma KB31; or (especially with regard to leukemias) K562 cells (American Type Culture Collection, Mannassas, VA) or human CFU-G cells (CFU-G stands for granulocyte colony forming unit, and it represents an early but commited granulocyte forming precursor cell that circulates in the blood stream or bone marrow) each of which is transplanted into female or male Balb/c nude mice.

Tumors or leukemias are obtained after subcutaneous injcetion of cells (minimum 2 x 10⁶ cells in 100 ml phosphate buffered physiological saline) into the carrier mice (e.g. 4-8 mice per cell line). The resulting tumors are passed serially through at least three subsequent transplantations before treatment is started. Tumor fragments (about 25 mg each) are injected s.c. into the left flank of the animals using a 13-gauge Trocar needle under Forene narcosis (Abbott, Switzerland) for implantation. All mice transplanted with estrogen-dependent tumor are, in addition, supplied with an estrogen pellet (1,0 cm of a tube with a quality appropriate for medical purposes, Dow Chemicals, with 5 mg estradiole, Sigma). The treatment ist started routinely (that is at low or intermediate tumor burden), as soon as the tumor has reached an average diameter of 100 mm³. Tumor growth is determined once, twice or thrice weekly (depending on tumor growth of the cell line) and 24 h after the last treatment by measurement of the perpendicular diameter. The tumor volumes are determined according to the formula L x D x p/6 (see Evans, B.D., Smith, I.E., Shorthouse, A.J. and Millar, J.J., Brit. J. Cancer, 45: 466-468, 1982). The antitumor activity is expressed as T/C% (average increase of the tumor volume of treated animals divided by the average increase of tumor volume in control animals multiplied by 100). Tumor regression (%) represents the smallest mean tumor volume compared to the mean tumor volume at the beginning of the treatment. Each animal in which the tumor reaches a diameter of more than 1,5 to 2 cm³ is sacrificed.

The components are adminstered according to known schedules. For example, component (a) is administered daily during 2 to 7 subsequent weeks either orally (p.o.) or intravenously (i.v.).

An exemplary (though not limiting) schedule for administration of component (b) is daily administration, e.g. for 5 days, and/or administration at days 1, 4 and 9, with eventual repetition after a certain time without treatment. Alternatively, treatment several times a day (e.g. 2 to 5 times) or treatment by continuous administration (e.g. infusion), e.g. at the time points indicated in the last sentence, are possible. Generally, administration is orally or parenterally.

The test compounds are preferably diluted in water or in sterile 0.9% saline.

All human tumor cell lines are obtained from the American Type Culture Collection (ATCC, Rockville, MD., USA) if not indicated otherwise and are cultivated in the suggested media with the corresponding additives (ATCC culture conditions), if not mentioned otherwise. The c-*sis*- and v-*sis*- transformed BALB/c 3T3 cells are obtained from Dr. C. Stiles (Dana Farber Cancer Institute, Boston, MA, USA). They are cultured in "Dulbecco's modified Eagle's medium" (DMEM), that is supplemented with 10 % calf serum and Hygromycin B in a concentration of 0.2 mg/ml or G418 in a concentration of 0.5mg/ml. BALB/c AMuLV A.6R.1 cells (ATCC) are kept in DMEM, supplemented with 10% fetal calf serum.

Leukemia burden is assessed by examining both peripheral white blood count and weight of spleen and thymus in tumored animals.

It can be shown by established test models and in particular those test models described herein that a COMBINATION OF THE INVENTION results in a more effective delay of progression or treatment of a bcr/abl-related disease compared to the effects observed with the single combination partners. The person skilled in the pertinent art is fully enabled to select a relevant test model to prove the hereinbefore and hereinafter mentioned therapeutic indications and beneficial effects. The pharmacological activity of a COMBINATION OF THE INVENTION may, for example, be demonstrated in a clinical study or in a test procedure as essentially described hereinafter.

Suitable clinical studies are, for example, open label non-randomized, dose escalation studies in patients with chronic myelogenic leukemia (CML). Such studies particularly are capable of demonstrating the synergism of the active ingredients of the COMBINATIONS OF THE INVENTION. The beneficial effects on proliferative diseases can be determined directly through the results of these studies or by changes in the study design which are known as such to a person skilled in the art. Such studies are, in particular, suitable to compare the effects of a monotherapy using the active ingredients and a COMBINATION OF THE INVENTION. Preferably, the combination partner (a) is administered with a fixed dose and the dose of the combination partner (b) is escalated until the Maximum Tolerated Dosage is reached. In a preferred embodiment of the study, each patient receives daily doses of the combination partner (a). The efficacy of the treatment for tumors can be determined in such studies, e.g., after 18 or 24 weeks by regular radiologic evaluation of the tumors every 6-16 weeks. For CML efficacy of the treatment can be determined in such studies, e.g. by testing blood and bone marrow for hematologic response, cytogenetic, and molecular response.

Alternatively, a placebo-controlled, double blind study can be used in order to prove the benefits of the COMBINATION OF THE INVENTION mentioned herein.

The term "delay of progression" as used herein means administration of the combination to patients being in a pre-stage or in an early phase of the disease to be treated, in which patients for example a pre-form of the corresponding disease is diagnosed or which patients are in a condition, e.g. during a medical treatment or a condition resulting from an accident, under which it is likely that a corresponding disease will develop.

The COMBINATION OF THE INVENTION can be a combined pharmaceutical preparation or a pharmaceutical composition.

An especial embodiment of this invention is represented by a pharmaceutical composition comprising a quantity, which is jointly therapeutically effective against a proliferative disease or acute or chronic transplant rejection comprising the COMBINATION OF THE INVENTION. In this composition, the combination partners (a) and (b) can be administered together, one after the other or separately in one combined unit dosage form or in two separate unit dosage forms. The unit dosage form may also be a fixed combination.

The pharmaceutical compositions for separate administration of the combination partners (a) and (b) and for the administration in a fixed combination, i.e. a single galenical compositions comprising at least two combination partners (a) and (b) (active ingredients), according to the invention can be prepared in a manner known per se and are those suitable for enteral, such as oral or rectal, and parenteral administration to mammals (warm-blooded animals), including man, comprising a therapeutically effective amount of at least one pharmacologically active combination partner alone or in combination with one or more pharmaceutically acceptable carries, especially suitable for enteral or parenteral application.

Novel pharmaceutical composition contain, for example, from about 10 % to about 100 %, preferably from about 20 % to about 60 %, of the active ingredients (about, whereever used in the present disclosure, preferably meaning ± 5 %, especially ± 0 %). Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In particular, a therapeutically effective amount of each of the combination partners of the COMBINATION OF THE INVENTION may be administered simultaneously or sequentially and in any order, and the components may be administered separately or as a fixed combination. For example, the method of delay of progression or treatment of a proliferative disease and/or acute or chronic transplant rejection according to the invention may comprise (i) administration of the combination partner (a) in free or pharmaceutically acceptable salt form and (ii) adminstration of a combination partner (b) in free or pharmaceutically acceptable salt form, simultaneously or sequentially in any order, in jointly therapeutically effective amounts, preferably in synergistically effective amounts, e.g. in daily dosages corresponding to the amounts described herein. The individual combination partners of the COMBINATION OF THE INVENTION can be administered separately at different times during the course of therapy or concurrently in divided or single combination forms. Furthermore, the term administering also encompasses the use of a pro-drug of a combination partner that convert *in vivo* to the combination partner as such. The instant invention is therefore to be understood as embracing all such regimes of simultaneous or alternating treatment and the term administering is to be interpreted accordingly.

The effective dosage of each of the combination partners employed in the COMBINATION OF THE INVENTION may vary depending on the particular compound or pharmaceutical composition employed, the mode of administration, the condition being treated, the severity of the condition being treated. Thus, the dosage regimen the COMBINATION OF THE INVENTION is selected in accordance with a variety of factors including the route of administration and the renal and hepatic function of the patient. A physician, clinician or veterinarian of ordinary skill can readily determine and prescribe the effective amount of the single active ingredients required to prevent, counter or arrest the progress of the condition. Optimal precision in achieving concentration of the active ingredients within the range that yields efficacy without toxicity requires a regimen based on the kinetics of the active ingredients' availability to target sites.

Component (a), especially N-{5-[4-(4-methyl-piperazino-methyl)-benzoylamido]-2-methylphenyl}-4-(3-pyridyl)-2-pyrimidine-amine monomesylate, is preferably administered to a war-blooded animal, especially a human, in a dosage in the range of about 2.5 to 850 mg/day, more preferably 5 to 600 mg/day and most preferably 20 to 300 mg/day. Unless stated otherwise herein, the compound is preferably administered from one to four times per day.

Component (b), especially Adaphostin, is preferably administered to a warm-blooded animal, especially a human in a dosage in the range of about 1 to 6000 mg/day, more preferably 25 to 5000 mg/day, most preferably 50 to 4000 mg/day. Unless stated otherwise herein, the compound is preferably administered from one to 5, especially from 1 to 4 times per day.

Unit dosage forms contain, in addition to possible carrier materials, components (a) or (b) or (in the case of fixed combinations) (a) and (b) in amounts equal to, half, a third or a quarter of the amounts given in the last two paragraphs.

Furthermore, the present invention pertains to the use of a COMBINATION OF THE INVENTION for the delay of progression or treatment of a bcr/abl-realated disease, preferably as defined above, and for the preparation of a medicament for the delay of progression or treatment of said disease.

In addition, the present invention provides a (commercial) product comprising (a) and (b) as defined above as active ingredients and optionally a pharmaceutically acceptable carrier material for simultaneous, chronically staggered or (less preferably) separate use, or any combination thereof.

Simultaneous means that both component (a) and (b) are administered at approximately the same time, preferably within less than a minute of time difference, especially directly at the same time.

Chronically staggered means that the compounds may be given separately in such time intervals that they preferably, in the human to be treated, still show a (preferably synergistic) interaction, that is, are jointly therapeutically effective. Whether this is the case, can inter alia be determined by following the blood levels, showing that both compounds are present in the blood of the human to be treated at least during certain time intervals.

Separate preferably means that the components (a) and (b) are administered such that no overlap of measurable blood levels of both compounds are present in an overlapping manner (at the same time).

Any combination thereof preferably means that the components (a) and (b) may be administered at one time point simultaneously, followed by administration of only one component at a later time point and subsequently the other component or the combination of both components at a still later time point, and the like.

In a preferred embodiment, the (commercial) product is a commercial package comprising as active ingredients COMBINATION OF THE INVENTION, together with instructions for its simultaneous, separate or sequential use, or any combination thereof, in the delay of progression or treatment of a bcr/abl-related disease.

The method according to the invention specifically relates to a method of treating a warm-blooded animal, especially a human, having a bcr/abl-related disease, especially as defined above, comprising administering to the animal a combination which comprises component (a), especially N-{5-[4-(4-methyl-piperazino-methyl)-benzoylamido]-2-methylphenyl}-4-(3-pyridyl)-2-pyrimidine-amine, and component (b), especially Adaphostin, in a quantity which is (especially jointly) therapeutically effective against said bcr/abl-related disease and in which the components can also be present in the form of their pharmaceutically acceptable salts.

### Examples:

The following Example illustrates the invention described above. The beneficial effects of the COMBINATION OF THE INVENTION can also be determined by other test models known as such to the person skilled in the pertinent art.

### Example 1: Effect of combination treatment with adaphostin and STI571 on K562 cell line

a) Material: Adaphostin is obtained as synthesized by the Drug Synthesis and Chemistry Branch, Division of Cancer Treatment and Diagnosis, National Cancer Institute (Bethesda, MD). STI571 is from Novartis Pharma AG (Basel, Switzerland) (see also WO 99/03854). The compounds are dissolved in dimethylsulfoxide and diluted 1:1000 into tissue culture medium.
b) Cells: K562 cells (American Type Culture Collection, Manassas, VA) are passaged in RPMI 1640 containing 5% heat-inactivated fetal bovine serum, 100 units/mL penicillin G, 100 µg/mL streptomycin and 2 mmol/L glutamine (medium A). ST1571-resistant K562 cells (see Weisberg et al., Blood 95, 3498-3505 (2000)) are maintained in medium A containing or lacking 0.5 µM STI571.
c) Clonogenic Assays: Aliquots containing 0.5x10⁶ K562 cells in 1 mL medium A are incubated with diluent, STI571 and/or adaphostin (see below) for the indicated lengths of time, sedimented at 100 x g for 5 min, diluted and plated in gridded 35-mm plates in the medium of Pike and Robinson containing 0.3% (w/v) Bacto agar. After incubation for 10-14 days at 37°C, colonies containing ≥50 cells are counted on an inverted phase contrast microscope.
   Clinical samples are studied under the aegis of a protocol approved by the Institutional Review Board of the Mayo Clinic in accordance with the policies of the US Department of Health and Human Services. To evaluate the effects of adaphostin and STI571 on normal vs. CML CFU-G, 10 mL of peripheral blood is drawn from normal volunteers and consenting patients with previously untreated chronic phase CML using EDTA as an anticoagulant. Mononuclear cells (d = 1.077 gm/cm³) harvested from ficoll-Hypaque step gradients are cultured for 24 h at a density of 1 x 10⁶/mL in Iscove's modified Dulbecco's medium containing 20% (v/v) FBS (medium C) supplemented with increasing concentrations of adaphostin or STI571. At the completion of the incubation, samples are sedimented at 200 x g for 10 min, resuspended at a concentration of 1 x 10⁶/ml in medium C containing 50 ng/mL G-CSF, plated in 0.3% agar, and incubated for 7-8 d. Colonies containing ≥32 cells are counted on an inverted phase contrast microscope.
d) Determination of Morphologic Changes Associated with Apoptosis:
   For morphological analysis, cells are fixed in 3:1 (v:v) methanol:acetic acid, stained with 1 µg/ml Hoechst 33258 in 50 % (v:v) glycerol and examined under epi-illumination using a Zeiss Axioplan microscope. Two hundred to 300 cells/sample are scored for apoptotic changes (peripheral chromatin condensed or nuclear fragmentation) as described in Svingen et al., Clin. Cancer Res. 6, 237-49 (2000).
e) Results:
   K562 cells are incubated for 24 h with the indicated concentration of adaphostin in the presence of a diluent or 20 µmol/l STI571. At the completion of the incubation, cells are washed, plated in 0.3 % agar and incubated for 10-14 days before colony formation is assessed. For results see under (a) in Table (A).

In an alternative experiment (see under (b) in Table (A)), cells are incubated 24 h with the indicated concentration of STI571 in the absence or presence of 5 µmol/l adaphostin. At the completion of the incubation, colony formation ability is assessed as described in the last paragraph.

Under (c) in Table (A), K562 cells are incubated with the concentration indicated in the subsequent table in the presence of diluent or 10 µmol/l STI 571, fixed, and examined for apoptotic morphological changes.

Under (d) in Table (A), circulating mononuclear cells from a CML patient are treated for 24 h with the indicated concentrations of adaphostin in the absence or presence of 20 µmol/l STI571, washed and plated in 0.3 % agar containing 50 ng/ml G-CSF. After 8 days, granulocyte colonies are counted.

Under (e) in Table (A), a summary of the results obtained when the experiment depicted under (d) is performed on six samples is presented.

Table (A):
(a) Effect of Adaphostin on Colony Formation of K562 Cells in the Absence and Presence of STI 572

| Adaphostin concentration (µmol/l) | without STI571 (concentration in µmol/l) | Number of colonies (% of control) | plus STI 571 (concentration in µmol/l) | Number of colonies (% of control) |
|---|---|---|---|---|
| 0 | 0 | 100 | 20 | 94±8 |
| 2.5 | 0 | 56±4 | 20 | 20±3 |
| 5.1 | 0 | 46±4 | 20 | 3.0±0.9 |
| 7.7 | 0 | 27±4 | 20 | 0.4±0.3 |
| 10.2 | 0 | 10±1 | 20 | 0.08±0.16 |

Data are expressed as 100 x the ratio of the number of colonies that form in 0.3 % agar after treatment with the indicated drugs to the number of colonies that form in 0.3 % agar after treatment with the diluent.
(b) Effect of Various STI571 Concentrations on Colony Formation of K562 Cells in the Absence and Presence of Adaphostin

| STI571 concentration (µmol/l) | without Adaphostin (concentration in µmol/l) | Number of colonies (% of control) | plus Adaphostin (concentration in µmol/l) | Number of colonies (% of control) |
|---|---|---|---|---|
| 0 | 0 | 100 | 5.2 | 35±3 |
| 1.2 | 0 | 83±1 | 5.2 | 30±7 |
| 2. | 0 | 98±8 | 5.2 | 22±2 |
| 5.0 | 0 | 85±8 | 5.2 | 16±2 |
| 10 | 0 | 72±5 | 5.2 | 9.7±1.7 |
| 20 | 0 | 76±4 | 5.2 | 4.5±1.7 |

Data are expressed as 100 x the ratio of the number of colonies that form in 0.3 % agar after treatment with the indicated drugs to the number of colonies that form in 0.3 % agar after treatment with the diluent.
(c) Effect of STI571 on Adaphostin-induced Apoptosis in K562 Cells

| Adaphostin concentration (µmol/l) | without ST1571 (concentration in µmol/l) | Apoptosis (% of total cells) | plus STI 571 (concentration in µmol/l) | Apoptosis (% of total cells) |
|---|---|---|---|---|
| 0 | 0 | 1.2 | 10 | 12.9 |
| 0.62 | 0 | 2.1 | 10 | 24.3 |
| 1.25 | 0 | 10.9 | 10 | 52.2 |
| 2.50 | 0 | 41.8 | 10 | 85.2 |
| 3.75 | 0 | 62.8 | 10 | 96.6 |
| 5.00 | 0 | 84.1 | 10 | 97.8 |

Data are expressed as percentage of morphologically apoptotic K562 cells observed after a 24 h treatment with the indicated concentration of adaphostin in the absence or presence of STI571.
(d) Effect of Adaphostin on Colony Formation of Pt. # 30 CFU-GM in the Absence and Presence of STI571

| Adaphostin concentration (µmol/l) | without STI571 (concentration in µmol/l) | Number of colonies (% of control) | plus STI 571 (concentration in µmol/l) | Number of colonies (% of control) |
|---|---|---|---|---|
| 0 | 0 | 100 | 20 | 98 |
| 3.2 | 0 | 93±1.9 | 20 | 87±1.8 |
| 6.4 | 0 | 71±2.2 | 20 | 80±2.0 |
| 12.7 | 0 | 55±2.5 | 20 | 25±0.3 |
| 15.9 | 0 | 40±1.2 | 20 | 13±2.0 |
| 19.1 | 0 | 18±0.7 | 20 | 5±0.9 |

Data are expressed as 100 x the ratio of the number of granulocyte colonies that form in 0.3 % agar after treatment with the indicated drugs to the number of colonies that form in 0.3 % agar after treatment with the diluent.
e) Effect of STI 71 on IC₅₀ of CFU-GM Exposed to Various Adaphostin Concentrations

| Patient number | IC₅₀ for Adaphostin without STI571 (µmol/l) | IC₅₀ for Adaphostin in presence of 20 µmol/l STI571 (µmol/l) |
|---|---|---|
| 28 | 13 | 5 |
| 29 | >20 | 14 |
| 30 | 13.5 | 8.5 |
| 31 | 8.5 | 10 |
| 33 | 23 | 16 |
| 34 | 11 | 3.5 |

The adaphostin concentration required to inhibit granulocyte formation by 50 % (IC₅₀) is determined from data similar to that in Table (A), (d) using linear regression.

Synergy between adaphostin and STI571: When parental K562 cells are treated with increasing concentrations of adaphostin, the addition of STI571 enhances the antiproliferative effects at all adaphostin concentrations analyzed (Table (A), (a)). This enhancement is evident at ST1571 concentrations as low as 2.5 µmol/l (Table (A), (b)). Further examination reveals that the enhanced inhibition of colony formation reflects induction of apoptosis in greater percentage of cells treated with combination as compared to cells treated with either drug alone (Table (A), (c)).

Additional experiments reveal that the effects of combining adaphostin and STI571 are not limited to K562 cells. Treatment with STI571 enhanced the antiproliferative effects of adaphostin on circulating CML myeloid precursors as well (Table (A), (d)). Similar effects are obtained in myeloid progenitros of 5 of 6 patients examined (Table (A), (e)).

In summary, the combination kills many more K562 cells than either agent alone (Table (A), (a), (c)). In addition, the combination inhibits outgrowth of CML CFU-G more than either agent alone (Table (A), (e), (f)).

## Claims

1. A combination of (a) an ATP-competitive inhibitor of Bcr/abl kinase activity and (b) a tyrphostin analog, wherein the ATP-competitive inhibitor of bcr/abl kinase activity (a) is (N-{5-[4-(4-methyl-piperazino-methyl)-benzoylamido]-2-methylphenyl}-4-(3-pyridyl)-2-pyrimidine-amine, and the tyrphostin analog (b) is Adaphostin, or a pharmaceutically acceptable salt of any one of components (a) or (b) where salt-forming groups are present.

2. A pharmaceutical preparation comprising (a) and (b) as mentioned in claim 1.

3. A product comprising (a) and (b) as defined in claim 1 and optionally a pharmaceutically acceptable carrier material, for simultaneous, chronically staggered and/or separate use, or any combination thereof.

4. The use of a combination according to claim 1 or a product according to claim 3 for the manufacture of a medicament for the treatment of a bcr/abl-positive proliferative disease, selected from cancer and tumour diseases.

5. A component (a), as defined in claim 1, for use in combination, that is at the same time point or in a chronologically staggered way, with a component (b) as defined in claim 1, or vice versa a component (b) for use in combination with a component (a) as defined hereinabove or hereinbelow, in the treatment of a bcr/abl-positive proliferative disease; especially said components in a packaging and with a description (e.g. package leaflet) suggesting or describing such combination.

## Patentansprüche

1. Kombination aus (a) einem ATP kompetitiven Inhibitor für eine BCR-ABL Kinaseaktivität und (b) einem Tyrphostinanalogon, worin der ATP kompetitive Inhibitor für eine BCR-ABL Kinaseaktivität (a) (N-{5-[4-(4-Methylpiperazinomethyl)-benzoylamido]-2-methylphenyl}-4-(3-pyridyl)-2-pyrimidinamin ist und das Tyrphostinanalogon (b) Adaphostin ist, oder ein pharmazeutisch akzeptables Salz einer der Komponenten (a) oder (b), sofern Salz bildende Gruppen vorhanden sind.

2. Pharmazeutische Präparation, umfassend (a) und (b) wie im Anspruch 1 erwähnt.

3. Produkt, umfassend (a) und (b) wie im Anspruch 1 definiert und optional ein pharmazeutisch akzeptables Trägermaterial, für eine simultane, chronologisch gestaffelte und/oder separate Anwendung, oder irgendeine Kombination hiervon.

4. Verwendung einer Kombination nach Anspruch 1 oder eines Produkts nach Anspruch 3 zur Herstellung eines Arzneimittels für die Behandlung einer BCR-ABL positiven proliferativen Krankheit, die ausgewählt ist aus einer Krebskrankheit und einer Tumorkrankheit.

5. Komponente (a) wie im Anspruch 1 definiert, zur Anwendung in Kombination, nämlich zum gleichen Zeitpunkt oder in einer chronologisch gestaffelten Weise, mit einer Komponente (b) wie im Anspruch 1 definiert, oder vice versa einer Komponente (b) zur Anwendung in Kombination mit einer Komponente (a) wie oben oder im Folgenden definiert, für die Behandlung einer BCR-ABL positiven proliferativen Krankheit, wobei diese Komponenten insbesondere in einer Packung angeordnet und mit einer Beschreibung versehen sind, beispielsweise mit einer Packungsbeilage, zwecks Hinweis auf oder Beschreibung für eine solche Kombination.

## Revendications

1. Combinaison de (a) un inhibiteur ATP compétitif de l'activité de la kinase Bcr/abl et (b) un analogue de tyrphostine, dans laquelle l'inhibiteur ATP compétitif de l'activité de la kinase Bcr/abl (a) est la (N-{5-[4-(4-méthyl-pipérazino-méthyl)-benzoylamido]-2-méthylphényl}-4-(3-pyridyl)-2-pyrimidine-amine, et l'analogue de tyrphostine (b) est l'Adaphostine, ou l'un quelconque des sels pharmaceutiquement acceptables des composants (a) ou (b) lorsque des groupes salificateurs sont présents.

2. Préparation pharmaceutique comprenant (a) et (b) tels que mentionnés dans la revendication 1.

3. Produit comprenant (a) et (b) tels que définis dans la revendication 1, et facultativement une matière support pharmaceutiquement acceptable, pour une utilisation simultanée, échelonnée chroniquement et/ou distincte, ou l'une quelconque de leurs combinaisons.

4. Utilisation d'une combinaison selon la revendication 1, ou d'un produit selon la revendication 3 pour la préparation d'un médicament pour le traitement d'une maladie proliférative bcr/abl-positive, choisie parmi des maladies cancéreuses et tumorales.

5. Composant (a), tel que défini dans la revendication 1, à utiliser en combinaison, c'est-à-dire au même point de temps, ou de manière chronologiquement échelonnée, avec un composant (b) tel que défini dans la revendication 1, ou *vice versa* composant (b) à utiliser en combinaison avec un composant (a) tel que défini ci-dessus ou ci-dessous, dans le traitement d'une maladie proliférative bcr/abl-positive ; en particulier, lesdits composants dans un conditionnement et avec une description (par exemple une notice d'information) suggérant ou décrivant une telle combinaison.
